# EUROPÄISCHE PATENTSCHRIFT

(11) **EP 4 011 333 B1**
(45) Veröffentlichungstag und Bekanntmachung des Hinweises auf die Patenterteilung: **06.08.2025**
(21) Anmeldenummer: 20213172.8
(22) Anmeldetag: 10.12.2020
(51) Int. Cl.: A61F 2/30, A61F 2/34, B33Y 80/00

(54) **HÜFTPFANNE EINER HÜFTGELENK-ENDOPROTHESE**
ACETABULAR CUP FOR A HIP JOINT ENDOPROSTHESIS
CUPULE ACETABULAIRE D'UNE ENDOPROTHÈSE DE L'ARTICULATION DE LA HANCHE

(43) Veröffentlichungstag der Anmeldung: 15.06.2022
(73) Patentinhaber: ARTIQO GmbH, 59348 Lüdinghausen (DE)
(72) Erfinder: Bücken, Ulrich, 59348 Lüdinghausen (DE); Frank, Thomas Mario, 7000 Eisenstadt (AT); Limberger, Tanja, 78166 Donaueschingen (DE)
(74) Vertreter: Gosdin, Carstensen & Partner Patentanwälte Partnerschaftgesellschaft mbB

(56) Entgegenhaltungen:
- EP-A1- 1 685 810
- EP-A1- 2 574 309
- CA-A1- 2 529 884
- DE-A1- 4 211 346
- US-A- 4 715 860
- US-A1- 2018 193 152
- US-A1- 2020 276 019
- US-B1- 6 682 567

## Beschreibung

Die Erfindung wird durch Anspruch 1 definiert und betrifft eine Hüftpfanne einer Hüftgelenk-Endoprothese, umfassend einen äußeren Pfannenabschnitt mit einer konvexen Oberfläche zur Kontaktnahme mit dem Acetabulum und mit einem inneren Pfannenabschnitt mit einer konkaven Oberfläche zur Kontaktnahme mit einem Inlay der Hüftgelenk-Endoprothese, wobei zumindest über einen Oberflächenabschnitt der Hüftpfanne der äußere Pfannenabschnitt relativ zum inneren Pfannenabschnitt in eine radiale Richtung federelastisch gestaltet ist, wobei die Hüftpfanne einen oberen Pol und ein vom Pol abgewandtes unteres Ende aufweist, wobei die Federelastizität durch einen Wandungsabschnitt des äußeren Pfannenabschnitts gebildet wird, der zu einem Wandungsabschnitt des inneren Pfannenabschnitts unter Bildung eines Hohlraums mit radialem Abstand verläuft, wobei der äußere Pfannenabschnitt und der innere Pfannenabschnitt sowie der Wandungsabschnitt des äußeren Pfannenabschnitts und der Wandungsabschnitt des inneren Pfannenabschnitts einstückig miteinander ausgebildet sind, wobei der Hohlraum frei von Federelementen ist, wobei der Hohlraum um den Umfang der Hüftpfanne vollständig umläuft und im Bereich des unteren Endes offen ist, wobei der äußere Pfannenabschnitt eine radial außenliegende poröse Struktur aufweist, an die sich eine radial innenliegende massive Struktur anschließt, wobei die außenliegende poröse Struktur und die innenliegende massive Struktur einstückig miteinander ausgebildet sind, wobei der Wandungsabschnitt des äußeren Pfannenabschnitts die radial außenliegende poröse Struktur aufweist und wobei diese an einen radial innenliegenden Abschnitt mit massiver Struktur anschließt.

Eine Hüftpfanne der gattungsgemäßen Art ist aus der EP 2 574 309 A1 bekannt. Hier ist vorgesehen, dass im doppelwandig ausgebildeten Bereich der Pfanne der innere Pfannenabschnitt zirkulär umläuft, während der äußere Pfannenabschnitt durch eine Anzahl an Segmenten gebildet wird, die in Umfangsrichtung durch Schlitze beanstandet sind. Somit kann jedes Segment unabhängig von einem anderen Segment lamellenartig radial einfedern und sich so an das Acetabulum anlegen.

Eine andere Hüftpfanne ist in der EP 3 714 840 A1 beschrieben. Die dort offenbarte Hüftpfanne ist in einem von einem Pol (d. h. vom oberen Ende) abgewandten unteren Ende über eine gewisse Höhe in radiale Richtung federelastisch ausgebildet. Hierfür sind nach einer Ausführungsform in einem geschlossenen Hohlraum Federelemente angeordnet, so dass der äußere Pfannenabschnitt relativ zum inneren Pfannenabschnitt in radialer Richtung elastisch ist. Eine weitere dort beschriebene Ausführungsform sieht zwei Schalenteile vor, die miteinander verbunden sind. Im vom Pol abgewandten unteren Ende hat das innere Schalenteil eine Anzahl Federlamellen, die das äußere Schalenteil abstützen, um so gezielt eine Elastizität zu erzeugen.

Weitere ähnliche Lösungen zeigen die EP 1 685 810 A1**,** die CA 2 529 884 A1**,** die US 6 682 567 B1 und die US 2020/276019 A1**.**

Die US 4 715 860 A offenbart eine Hüftpfanne einer ähnlichen Art, bei der im unteren Bereich ein Metallring vorgesehen ist, der das Lösen porösen Materials beim Einpressen der Hüftpfanne in das Acetabulum verhindern soll. Die Hüftpfanne ist allerdings in diesem Falle nicht mit einer Struktur versehen, welche sie wie im Falle der EP 2 574 309 A1 federelastisch macht. Dies gilt auch hinsichtlich der DE 42 11 346 A1, die eine Hüftpfanne mit im Wesentlichen massiver Struktur offenbart, die an der Außenseite mit einer offenzelligen Oberflächenstruktur versehen ist.

Im Englischen wird die Hüftpfanne als "cup" oder "shell" bezeichnet und besteht zumeist aus Metall. Sie wird in das bzw. anstelle des Acetabulums, d. h. der natürlichen Hüftpfanne des Beckenknochens, implantiert, wobei der Aufnahmebereich für die Hüftpfanne kugelabschnittsartig vorgefräst wird. Der äußere Pfannenabschnitt mit seiner konvexen Oberfläche ist demgemäß im implantierten Zustand im Kontakt mit dem Beckenknochen, während der innere Pfannenabschnitt mit seiner (im Wesentlichen) konkaven Fläche einen Aufnahmeraum für ein Inlay der Prothese bildet.

Das Inlay wird in die Konkavität der zumeist metallischen Hüftpfanne eingesetzt und mit dieser über eine Inlaykupplung oder Innenkupplung kraft- oder formschlüssig verbunden. Es nimmt den metallischen oder keramischen Hüftkopf des femoralen Teils der Hüftprothese auf.

Dabei ist vorgesehen, dass die Prothesen-Hüftpfanne mit Übermaß ("Pressfit") in das Acetabulum implantiert, d. h. in dieses eingeschlagen wird. Daher besitzen üblicherweise zu implantierende Hüftpfannen im Durchmesser ein Übermaß von ca. 2 % bis 4 % gegenüber dem gefrästen Acetabulum. Dies bedeutet, dass beispielweise eine Hüftpfanne mit einer Größe von 50 mm einen effektiven Durchmesser von beispielsweise 51 mm bzw. 52 mm besitzt.

Demgemäß wird durch das Übermaß beim Einschlagen der Hüftpfanne eine gewünschte Vorspannung (d. h. das "Pressfit") erzeugt. Die zumeist metallische Hüftpfanne soll sich beim Einschlagen im knöchernen, ausgefrästen Acetabulum möglichst gleichmäßig verspannen, ohne dass an der Innenkontur ein nennenswerter Verzug, beispielsweise eine ovale Entrundung, eintritt.

Das Pressfit ist gemeinsam mit einer möglichst rauen (konvexen äußeren) Oberfläche der Hüftpfanne für die Primärstabilität der künstliche Hüftpfanne verantwortlich. Das Pressfit ist im Bereich des Pfannenrandes am stärksten und nimmt zum Pfannenboden (d. h. zum Pol) hin ab.

Die Sekundärstabilität der Hüftpfanne wird nach 6 bis 12 Wochen durch das Einwachsen von Knochen in die raue Oberfläche erreicht, gleichzeitig wird die Vorspannung (d. h. das Pressfit) durch Umbauvorgänge des Knochens abgebaut.

Bekannt ist es, dass Pressfitpfannen modular aufgebaut sind. Dies bedeutet, dass in die metallische Hüftpfanne (mittels einer konischen oder sphärischen Kupplung) Inlays aus Kunststoff und/oder Keramik eingebracht werden können. Die konischen Innenkupplungen unterliegen (vor allem für die Aufnahme von keramischen Inlays) hohen Genauigkeiten in Bezug auf Winkeltoleranzen, Durchmesser und Rundheit.

Da die Inlays nach dem Einschlagen in die Pfanne eingesetzt werden (es besteht eine entsprechende Auswahlmöglichkeit verschiedener Inlaytypen je nach intraoperativer Situation), darf der Einschlagvorgang einschließlich des Pressfits die geforderte Genauigkeit der Inlaykupplung nicht beeinträchtigen. Gleichzeit ist beim Einschlagen der Hüftpfanne ein gutes Setzverhalten derselben gefordert. Dies wird (neben der Außengeometrie) von der Wandstärke der Hüftpfannen-Schale beeinflusst.

Dünnwandige Pfannen mit einem höheren elastischen Verhalten der Schale (Federwirkung) erreichen durch Verformung das angestrebte günstigere Setzverhalten gegenüber dickwandigen bzw. steifen Pfannen. Unterschiede in der Knochenhärte können auch besser toleriert werden.

Ein Sortiment von Pressfitpfannen umfasst meist zehn Größen von Durchmesser 46 bis 64. Um das Inlaysortiment so klein wie möglich zu halten, werden oft zwei oder mehrere Hüftpfannen-Kopfgrößen mit einer äußeren Inlaykontur ausgestattet. Dies führt zu variierenden Wandstärken innerhalb eines Pfannensystems, so dass sich ein größenspezifisches Einschlagverhalten ergibt. Des Weiteren haben verschiedene Pfannensteifigkeiten in Zusammenhang mit variierenden Durchmessern einen Einfluss auf die Spannung im umgebenden Knochen und eventuell auf die Umbauvorgänge des Knochengewebes.

Unter den beschriebenen Gesichtspunkten liefert die oben beschriebene vorbekannte Lösung bereits gute Ergebnisse.

Der vorliegenden Erfindung liegt die **Aufgabe** zugrunde, eine Hüftpfanne der eingangs genannten Art so weiterzubilden, dass diese ein weiter verbessertes einheitliches Setz- bzw. Einschlagverhalten und eine Formstabilität des konkaven inneren Pfannenabschnitts über das gesamte zum Einsatz kommende Größenspektrum aufweist. Demgemäß soll sich eine beim Setzen und/oder Einschlagen erfolgende Formänderung des äußeren Pfannenabschnitts nicht oder zumindest möglichst wenig auf die Kontur des inneren Pfannenabschnitts übertragen. Gegebenenfalls vorhandene Ovalitäten im Acetabulum sollen verbessert ausgeglichen werden können.

Die **Lösung** dieser Aufgabe durch die Erfindung ist dadurch gekennzeichnet, dass der radial innenliegende Abschnitt mit massiver Struktur im Bereich des unteren Endes im Schnitt eine L-förmige Gestalt aufweist, mit der die radial außenliegende poröse Struktur im Bereich des unteren Endes abgeschlossen wird.

Im Unterschied zu der vorbekannten Lösung wird also auf eine einstückige Gestaltung der Hüftpfanne abgestellt, wobei sich das untere Ende (in einem Radialschnitt gesehen) gabelförmig öffnet und einen freien bzw. leeren Hohlraum zwischen äußerem und innerem Pfannenabschnitt bildet, wobei die genannte weitere L-förmige Gestaltung der massiven Struktur vorgesehen ist.

Die poröse Struktur kann dabei durch ein Gitter, insbesondere durch ein kubisches Gitter, gebildet werden.

Zwecks weiterer Verbesserung des Setz- bzw. Einschlagverhalten kann hierbei vorgesehen werden, dass die radial außenliegende poröse Struktur eine radiale Dicke aufweist und dass der radial innenliegende Abschnitt mit massiver Struktur eine radiale Dicke aufweist, wobei die radiale Dicke der massiven Struktur im Bereich des unteren Endes zwischen 30 % und 50 % der gesamten radialen Dicke von poröser und massiver Struktur beträgt.

Weiterhin weist der radial innenliegende Abschnitt mit massiver Struktur am vom unteren Ende abgewandten Ende des Hohlraums eine Dicke (gemessen senkrecht zur Mittenachse der Hüftpfanne) auf, die bevorzugt zwischen 0,8 mm und 1,25 mm beträgt.

Die genannte poröse Struktur hat bevorzugt eine Porösität von mindestens 65 %, vorzugsweise von mindestens 85 % (hierunter ist die dimensionslose Größe zu verstehen, die das Verhältnis des Hohlraumvolumens zum Gesamtvolumen der porösen Struktur beschreibt; hier als prozentuale Größe angegeben).

Im Bereich des Oberflächenabschnitts weist der Wandungsabschnitt des äußeren Pfannenabschnitts eine radiale Dicke auf, während der Wandungsabschnitt des inneren Pfannenabschnitts eine radiale Dicke aufweist, wobei die radiale Dicke des Wandungsabschnitts des inneren Pfannenabschnitts im Bereich des unteren Endes bevorzugt zwischen 150 % und 250 % der radialen Dicke des Wandungsabschnitts des äußeren Pfannenabschnitts beträgt.

Ferner ist bevorzugt vorgesehen, dass im lastfreien Zustand der Hüftpfanne im Bereich des Oberflächenabschnitts zwischen dem Wandungsabschnitt des äußeren Pfannenabschnitts und dem Wandungsabschnitt des inneren Pfannenabschnitts der Hohlraum mit einer radialen Dicke vorliegt, wobei die radiale Dicke des Hohlraums im Bereich des unteren Endes zwischen 5 % und 30 % der radialen Dicke des Wandungsabschnitts des inneren Pfannenabschnitts beträgt.

Der Oberflächenabschnitt mit dem Wandungsabschnitt des äußeren Pfannenabschnitts und dem Wandungsabschnitt des inneren Pfannenabschnitts erstreckt sich vom unteren Ende aus bevorzugt über einen Winkel, der zwischen 25° und 35° beträgt. Ein besonders bevorzugter Wertebereich für den Winkel liegt dabei zwischen 28° und 32°.

Die Länge des Hohlraums (gemessen in Richtung der Mittenachse) beträgt bevorzugt zwischen 6,0 mm und 15,0 mm, besonders bevorzugt zwischen 8,5 mm und 13,5 mm.

Weiterhin kann vorgesehen werden, dass die radiale Dicke des massiven Abschnitts des äußeren Pfannenabschnitts beim Fortschreiten von seinem dem Pol zugewandten Ende in Richtung zum unteren Ende abnimmt.

Die Hüftpfanne besteht bevorzugt aus Metall.

Dabei ist insbesondere vorgesehen, dass sie durch einen additiven Fertigungsprozess hergestellt ist. Hierunter ist insbesondere, aber nicht ausschließlich, der 3-D-Druck zu verstehen, mit dem es in besonders vorteilhafter Weise möglich ist, die gesamte Struktur der Hüftpfanne einteilig zu fertigen.

Durch die Wahl zumindest eines Teils der genannten Abmessungen bzw. Parameter ist es in besonders vorteilhafter Weise möglich, in effektiver Weise die Federkonstante der Hüftpfanne zu beeinflussen (wenn diese im Bereich des unteren Endes an diametral gegenüber liegenden Stellen mit einer Kraft beaufschlagt wird, welche zu einem radialen Einfedern führt). Damit kann im Sinne der oben genannten Aufgabenstellung eine Verbesserung des Einschlagverhaltens der Hüftpfanne in das Acetabulum sowie ein optimales Setzverhalten erreicht werden. Der radial außen liegende Bereich der Hüftpfanne weist somit einen gewünschten Grad an Nachgiebigkeit auf, während der radial innen liegende Bereich der Hüftpfanne eine hohe Formstabilität und somit eine stabile Innenkontur aufweist.

Damit ist eine günstige Federwirkung der Hüftpfanne gegeben, so dass ein gewünschtes Maß an Pressfit-Wirkung erreicht werden kann. Die Spannung im Knochenlager wird über das gesamte Größenspektrum homogenisiert. Das Innere der Hüftpfanne ist optimal für die Aufnahme von modularen Inlays geeignet. Somit sind die Anforderungen für Keramik- oder PE-Inlays erfüllt.

Die beim Einschlagen der Hüftpfanne in das Acetabulum entstehenden Spannungen wirken nicht wesentlich auf die Innenkontur, so dass die Verformungen hier gering bleiben. Gleichermaßen werden Spannungsspitzen vermieden bzw. reduziert, welche in situ beispielsweise beim Laufen oder Stolpern auftreten können; jedenfalls ist deren Wirkung auf die InlayKupplung reduziert.

Weiterhin ist es günstig, dass bei der Bereitstellung eines Sets von Hüftpfannen unterschiedlicher Größe eine Gestaltung mit relativ gleichbleibender Steifigkeit möglich ist.

In der Zeichnung ist ein Ausführungsbeispiel der Erfindung dargestellt. Es zeigen:
- Fig. 1: in einer Radialschnittdarstellung eine in ein nicht dargestelltes Acetabulum (Beckenpfanne) zu implantierende Hüftpfanne, die Bestandteil einer Hüftgelenk-Endoprothese ist,
- Fig. 2: eine vergrößerte Darstellung des rechten unteren Bereichs der Figur 1 mit Angabe einiger geometrischer Größen und
- Fig. 3: Vergleiche der Deformation des äußeren Bereichs der Hüftpfanne relativ zur Deformation des inneren Bereichs der Hüftpfanne bei Beaufschlagung mit einer Kraft, wobei zwei vorbekannte Lösungen sowie eine erfindungsgemäße Lösung dargestellt sind.

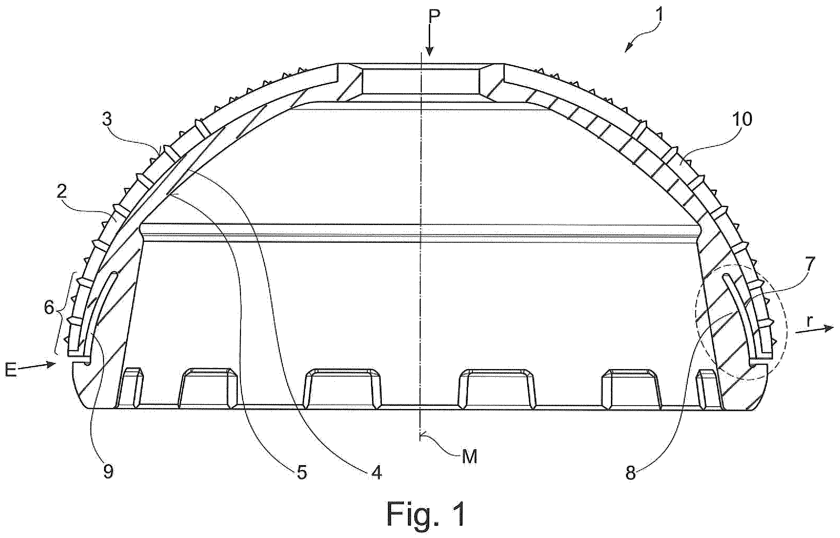

In Figur 1 ist eine Hüftpfanne 1 dargestellt, die in ein (nicht dargestelltes) Acetabulum implantiert wird. Die Hüftpfanne 1 ist Teil einer Hüftgelenk-Endoprothese.

Die Hüftpfanne 1 hat einen äußeren Pfannenabschnitt 2, der eine konvexe Oberfläche 3 hat. Diese kontaktiert im implantierten Zustand das Acetabulum. Weiterhin hat die Hüftpfanne 1 einen inneren Pfannenabschnitt 4, der im Wesentlichen eine konkave Oberfläche 5 hat und damit einen in etwa halbkugelförmigen Raum umschließt, in dem ein (nicht dargestelltes) Inlay der Hüftgelenk-Endoprothese angeordnet wird. Insoweit hat die Hüftpfanne 1 einen oberen Pol P sowie ein unteres Ende E, auf welche nachfolgend Bezug genommen wird. Weiterhin hat sie eine Mittenachse M.

In Figur 1 ist ein Radialschnitt durch die Hüftpfanne 1 dargestellt, d. h. ein Schnitt durch die Hüftpfanne 1 entlang eines Großkreises der halbkugelartigen Struktur der Hüftpfanne 1. Demgemäß ergibt sich eine radiale Richtung r von einem gedachten Mittelpunkt der halbkugelartigen Struktur der Hüftpfanne 1.

Am Pol P der Hüftpfanne 1 ist nach dem Einschlagen zumeist noch etwas Spiel vorhanden, da postoperativ mit Aufnahme der vollen Belastung eine gewisse Setzung der Hüftpfanne 1 zu erwarten ist.

Beim Einschlagvorgang ist nun angestrebt, dass sich lediglich die laterale Außenschale (Wandungsabschnitt 7 des äußeren Pfannenabschnitt 2, s. unten) verformt, während die mediale Innenschale (Wandungsabschnitt 8 des inneren Pfannenabschnitt 4, s. unten) sich möglichst wenig verformt.

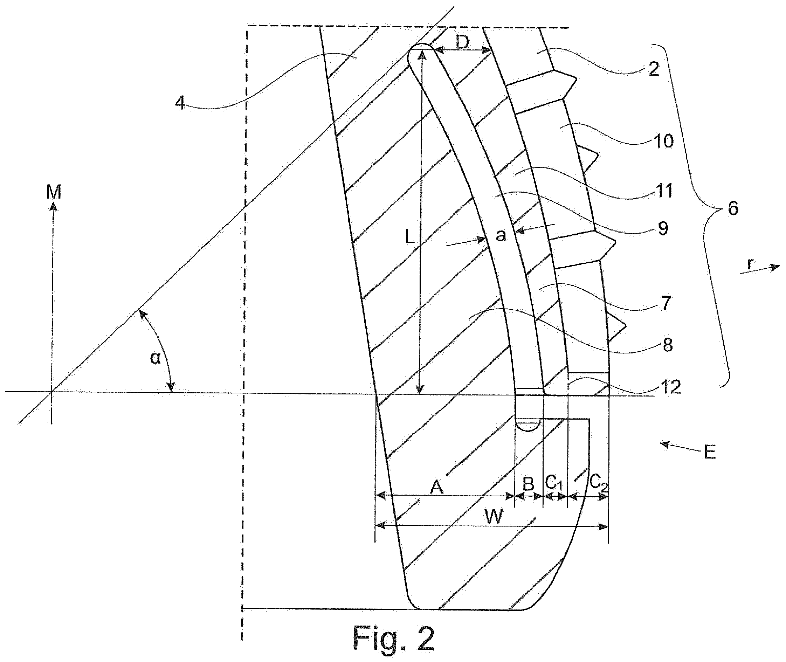

Um dies zu erreichen, ist vorgesehen, dass über einen Oberflächenabschnitt 6 der Hüftpfanne 1 der äußere Pfannenabschnitt 2 relativ zum inneren Pfannenabschnitt 4 in radiale Richtung r federelastisch gestaltet ist. Wie insbesondere in der Zusammenschau der Figuren 1 und 2 gesehen werden kann, ist eine federelastische Struktur über den Oberflächenabschnitt 6 gegeben; in diesem Bereich ist der Wandungsabschnitt 7 des äußeren Pfannenabschnitts 2 vom Wandungsabschnitt 8 des inneren Pfannenabschnitts 4 durch einen (zirkulär umlaufenden) Hohlraum 9 getrennt. Für den Hohlraum 9 ist ein radialer Abstand a in Figur 2 eingetragen, der seine radiale Erstreckung illustriert.

Dabei ist konkret erfindungsgemäß vorgesehen, dass der Hohlraum 9 um den Umfang der Hüftpfanne 1 vollständig umläuft und im Bereich des unteren Endes E offen ist (s. am besten Figur 2). Ferner sind der äußere Pfannenabschnitt 2 und der innere Pfannenabschnitt 4 sowie der Wandungsabschnitt 7 des äußeren Pfannenabschnitts 2 und der Wandungsabschnitt 8 des inneren Pfannenabschnitts 4 einstückig miteinander ausgebildet. Schließlich ist es wesentlich, dass der Hohlraum 9 frei von Federelementen ist.

Wie in den Figuren gesehen werden kann, ist dabei praktisch die gesamte radial außen liegende Oberfläche der Hüftpfanne 1 mit einer porösen Struktur versehen, während der radial innen liegende Bereich der Hüftpfanne 1 eine massive Struktur aufweist.

Dies bedeutet mit Blick auf den federelastisch ausgebildeten Bereich, dass der Wandungsabschnitt 7 des äußeren Pfannenabschnitts sowohl den radial außen liegenden porösen Abschnitt 10 als auch den radial innen liegenden massiven Abschnitt 11 aufweist, die miteinander einstückig ausgebildet sind.

Am besten geht aus Figur 2 hervor, dass dabei im Bereich des unteren Endes E der massive Abschnitt 11 (im dargestellten Radialschnitt) L-förmig ausgebildet ist und den porösen Abschnitt 10 nach unten abschließt. Die L-förmige Gestalt ist in Figur 2 mit der Bezugsziffer 12 versehen.

In Figur 2 sind dabei diverse geometrische Größen eingetragen, die die Gestaltung der Hüftpfanne 1 im Oberflächenabschnitt 6 definieren. Die oben angegebenen Wertebereiche für diese Größen bzw. Parameter haben sich als sehr vorteilhaft erwiesen, um das Einschlag- und Setzverhalten der Hüftpfanne 1 zu optimieren. Allerdings ist es nicht erforderlich, dass sämtliche Wertbereiche zwingend eingehalten werden müssen, um eine gute Lösung zu realisieren.

Zunächst ist der Winkel α in Figur 2 eingetragen, der angibt, über welche Winkelerstreckung (ausgehend vom unteren Ende des Wandungsabschnitt 7 des äußeren Pfannenabschnitts 2) sich der Hohlraum 9 ausdehnt. Ein Wertebereich für den Winkel α zwischen 28° und 32° ist besonders bevorzugt.

Eingetragen ist auch die Länge L des Hohlraums 9, wie sie sich, gemessen in Richtung der Mittenachse M, ergibt.

In Figur 2 sind weiterhin
- die radiale Dicke A des Wandungsabschnitts 8 des inneren Pfannenabschnitts 4,
- die radiale Dicke B des Hohlraums 9,
- die radiale Dicke C1 der radial innenliegende massiven Struktur 11 des Wandungsabschnitts 7 des äußeren Pfannenabschnitts 2,
- die radiale Dicke C2 der radial außenliegenden porösen Struktur 10 des Wandungsabschnitts 7 des äußeren Pfannenabschnitts 2,
- die Dicke D der der radial innenliegende massiven Struktur 11 des Wandungsabschnitts 7 des äußeren Pfannenabschnitts 2, sowie
- die gesamte radiale Weite W der Hüftpfannenwand
eingetragen, jeweils gemessen am unteren Ende des Wandungsabschnitt 7 des äußeren Pfannenabschnitts 2, d. h. im Bereich des unteren Endes E der Hüftpfanne 1.

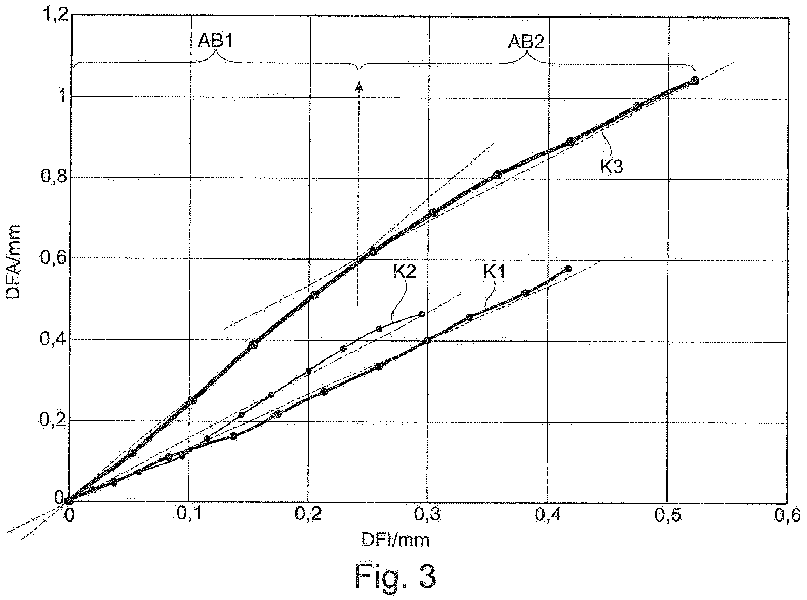

Der sich vorteilhaft ergebende Effekt gemäß der vorgeschlagenen Ausgestaltung der Hüftpfanne 1 ist in Figur 3 illustriert. Dargestellt ist hier die innere Deformation DFI in mm, aufgetragen über der äußeren Deformation DFA in mm. Demgemäß gibt das dargestellte Diagramm in Figur 3 das Verhältnis der Deformationen zwischen Außenkontur und Innenkontur der Hüftpfanne an, wenn diese einer Deformationskraft unterliegt. Es handelt sich hierbei um den Deformationstest nach ISO 7206-12. Die Hüftpfanne wird dabei einer diametral entgegengesetzten Zweipunktbelastung ausgesetzt.

Dargestellt ist mit K1 und K2 jeweils der Verlauf für zwei vorbekannte Hüftpfannen, während mit K3 der Verlauf für eine erfindungsgemäße Hüftpfanne dargestellt ist.

Durch die mit gestrichelten Linien eingetragenen Verläufe der Kurven K1 und K2 lässt sich sofort feststellen, dass ein weitgehend lineares Verhalten gegeben ist, wobei die Verformungen von außen nach innen im Wesentlichen direkt weitergegeben werden.

Für den Verlauf der Kurve K3 ist dies nicht der Fall. So hat eine äußere Verformung DFA von beispielsweise 0,4 mm nur eine innere Verformung DFI von ca. 0,16 mm zur Folge. Zu beachten ist auch, dass durch das erfindungsgemäß vorgeschlagene Konzept sich der Verlauf der Kurve K3 im Wesentlichen in zwei Abschnitte unterteilt:
In einem ersten Abschnitt AB1 liegt der Wandungsabschnitt 7 des äußeren Pfannenabschnitts 2 im Bereich des unteren Endes E noch nicht am Wandungsabschnitt 8 des inneren Pfannenabschnitts 4 an, d. h. es liegt noch ein freitragender Bereich vor.

Erst nach weiterer Verformung, nämlich im zweiten Abschnitt AB2, liegt der Wandungsabschnitt 7 am Wandungsabschnitt 8 im Bereich des unteren Endes E an, so dass jetzt das Verhältnis DFA / DFI einen anderen Verlauf nimmt. Eingezeichnet sind wiederum für beide Abschnitte AB1 und AB2 gestrichelte Linien, aus denen die Veränderung der Steigung der Kurve K3 am besagten Übergang (von AB1 zu AB2) ersichtlich ist.

Die vorgeschlagene Hüftpfanne 1 zeichnet sich demgemäß (unter anderem) dadurch aus, dass bei der Beaufschlagung mit einer radial wirkenden äußeren Deformationskraft an zwei diametral gegenüber liegenden Stellen im Bereich des unteren Endes E das Verhältnis der äußeren Deformation (DFA) des Wandungsabschnitt 7 des äußeren Pfannenabschnitts 2 zur inneren Deformation (DFI) des Wandungsabschnitt 8 des inneren Pfannenabschnitts 4 zunächst innerhalb eines ersten Abschnitts AB1 auf einem ersten Niveau liegt, um sich dann in einem sich anschließenden zweiten Abschnitt AB2 auf ein zweites Niveau zu ändern.

Somit kann beim Einschlagen der Hüftpfanne in das Acetabulum zunächst ein höheres Maß an Elastizität des Wandungsabschnitts 7 genutzt werden, ohne dass der Wandungsabschnitt 8 zu stark an der Verformung teilnimmt. Erst wenn der gegebene Federweg des Wandungsabschnitts 7 ausgenutzt ist und am unteren Ende E der Wandungsabschnitt 7 am Wandungsabschnitt 8 anliegt, ändert sich die Kennlinie, d. h. das der Verlauf des Verhältnisses DFA/DFI.

Die zum Einsatz kommenden Inlays sind zumeist modular gestaffelt und werden in verschiedenen Größenabstufungen vorgehalten. Hierbei sind unterschiedliche Hüftpfannen-Größen relevant, die das Inlay aufnehmen.

Weiterhin können verschiedene Außendurchmesser bzw. Außenkonturen vorgesehen sein, die exakt die Innendurchmesser bzw. Innenkontur der aufnehmenden metallischen Hüftpfanne passen müssen. Bei den Inlays werden üblicherweise mehrere Hüftkopf-Aufnahmedurchmesser mit einer identischen Außenkontur ausgestattet, so dass sie in ein und dieselbe metallische Hüftpfanne passen. Daraus ergeben sich dann natürlich entsprechende unterschiedliche Wandstärken des Inlays.

Betreffend die Hüftpfanne und das Inlay sind verschiedene Werkstoffkombinationen möglich. Das Inlay kann aus PE-Kunststoff oder Keramik bestehen. Folgende Materialpaarungen sind möglich bzw. gebräuchlich: Metall auf Polyäthylen (englisch abgekürzt "MoP"), Metall auf Keramik "MoC"), Keramik auf Polyäthylen ("CoP") und Keramik auf Keramik ("CoC").

### Bezugszeichenliste:

- 1: Hüftpfanne
- 2: äußerer Pfannenabschnitt
- 3: konvexe Oberfläche
- 4: innerer Pfannenabschnitt
- 5: konkave Oberfläche
- 6: Oberflächenabschnitt
- 7: Wandungsabschnitt des äußeren Pfannenabschnitts
- 8: Wandungsabschnitt des inneren Pfannenabschnitts
- 9: Hohlraum
- 10: poröse Struktur / poröser Abschnitt
- 11: massive Struktur / massiver Abschnitt
- 12: L-förmige Gestalt

- a: radialer Abstand
- r: radiale Richtung

- P: oberer Pol der Hüftpfanne
- E: unteres Ende der Hüftpfanne
- M: Mittenachse

- A: radiale Dicke des Wandungsabschnitts 8 des inneren Pfannenabschnitts

- B: radiale Dicke des Hohlraums 9

- C1: radiale Dicke der radial innenliegenden massiven Struktur des Wandungsabschnitts 7 des äußeren Pfannenabschnitts
- C2: radiale Dicke der radial außenliegenden porösen Struktur des Wandungsabschnitts 7 des äußeren Pfannenabschnitts
- C1 + C2: radiale Dicke der massiven und porösen Struktur

- D: Dicke der radial innenliegenden massiven Struktur des Wandungsabschnitts 7 des äußeren Pfannenabschnitts

- L: Länge des Hohlraums

- W: gesamte radiale Weite der Hüftpfannenwand

- α: Winkel

- DFI: Innere Deformation
- DFA: Äußere Deformation

## Patentansprüche

1. Hüftpfanne (1) einer Hüftgelenk-Endoprothese, umfassend einen äußeren Pfannenabschnitt (2) mit einer konvexen Oberfläche (3) zur Kontaktnahme mit dem Acetabulum und mit einem inneren Pfannenabschnitt (4) mit einer konkaven Oberfläche (5) zur Kontaktnahme mit einem Inlay der Hüftgelenk-Endoprothese, wobei zumindest über einen Oberflächenabschnitt (6) der Hüftpfanne (1) der äußere Pfannenabschnitt (2) relativ zum inneren Pfannenabschnitt (4) in eine radiale Richtung (r) federelastisch gestaltet ist, wobei die Hüftpfanne (1) einen oberen Pol (P) und ein vom Pol (P) abgewandtes unteres Ende (E) aufweist, wobei die Federelastizität durch einen Wandungsabschnitt (7) des äußeren Pfannenabschnitts (2) gebildet wird, der zu einem Wandungsabschnitt (8) des inneren Pfannenabschnitts (4) unter Bildung eines Hohlraums (9) mit radialem Abstand (a) verläuft, wobei der äußere Pfannenabschnitt (2) und der innere Pfannenabschnitt (4) sowie der Wandungsabschnitt (7) des äußeren Pfannenabschnitts (2) und der Wandungsabschnitt (8) des inneren Pfannenabschnitts (4) einstückig miteinander ausgebildet sind, wobei der Hohlraum (9) frei von Federelementen ist, wobei der Hohlraum (9) um den Umfang der Hüftpfanne (1) vollständig umläuft und im Bereich des unteren Endes (E) offen ist, wobei der äußere Pfannenabschnitt (2) eine radial außenliegende poröse Struktur (10) aufweist, an die sich eine radial innenliegende massive Struktur (11) anschließt, wobei die außenliegende poröse Struktur (10) und die innenliegende massive Struktur (11) einstückig miteinander ausgebildet sind, wobei der Wandungsabschnitt (7) des äußeren Pfannenabschnitts (2) die radial außenliegende poröse Struktur (10) aufweist und wobei diese an einen radial innenliegenden Abschnitt mit massiver Struktur (11) anschließt,
**dadurch gekennzeichnet,**
**dass** der radial innenliegende Abschnitt mit massiver Struktur (11) im Bereich des unteren Endes (E) im Schnitt eine L-förmige Gestalt (12) aufweist, mit der die radial außenliegende poröse Struktur (10) im Bereich des unteren Endes (E) abgeschlossen wird.

2. Hüftpfanne nach Anspruch 1, **dadurch gekennzeichnet, dass** die poröse Struktur (10) durch ein Gitter, insbesondere durch ein kubisches Gitter, gebildet wird.

3. Hüftpfanne nach Anspruch 1 oder 2, **dadurch gekennzeichnet, dass** die radial außenliegende poröse Struktur (10) eine radiale Dicke (C2) aufweist und dass der radial innenliegende Abschnitt mit massiver Struktur (11) eine radiale Dicke (C1) aufweist, wobei die radiale Dicke (C1) der massiven Struktur (11) im Bereich des unteren Endes (E) zwischen 30 % und 50 % der gesamten radialen Dicke von poröser und massiver Struktur beträgt.

4. Hüftpfanne nach einem der Ansprüche 1 bis 3, **dadurch gekennzeichnet, dass** der radial innenliegende Abschnitt mit massiver Struktur (11) am vom unteren Ende (E) abgewandten Ende des Hohlraums (9) eine Dicke (D), gemessen senkrecht zur Mittenachse (M), aufweist, die zwischen 0,8 mm und 1,25 mm beträgt.

5. Hüftpfanne nach einem der Ansprüche 1 bis 4, **dadurch gekennzeichnet, dass** die poröse Struktur (10) eine Porösität von mindestens 65 % aufweist, vorzugsweise von mindestens 85 %.

6. Hüftpfanne nach einem der Ansprüche 1 bis 5, **dadurch gekennzeichnet, dass** im Bereich des Oberflächenabschnitts (6) der Wandungsabschnitt (7) des äußeren Pfannenabschnitts (2) eine radiale Dicke aufweist und dass der Wandungsabschnitt (8) des inneren Pfannenabschnitts (4) eine radiale Dicke (A) aufweist, wobei die radiale Dicke (A) des Wandungsabschnitts (8) des inneren Pfannenabschnitts (4) im Bereich des unteren Endes (E) zwischen 150 % und 250 % der radialen Dicke des Wandungsabschnitts (7) des äußeren Pfannenabschnitts (2) beträgt.

7. Hüftpfanne nach einem der Ansprüche 1 bis 6, **dadurch gekennzeichnet, dass** im lastfreien Zustand der Hüftpfanne (1) im Bereich des Oberflächenabschnitts (6) zwischen dem Wandungsabschnitt (7) des äußeren Pfannenabschnitts (2) und dem Wandungsabschnitt (8) des inneren Pfannenabschnitts (4) der Hohlraum (9) mit einer radialen Dicke (B) vorliegt, wobei die radiale Dicke (B) des Hohlraums (9) im Bereich des unteren Endes (E) zwischen 5 % und 30 % der radialen Dicke (A) des Wandungsabschnitts (8) des inneren Pfannenabschnitts (4) beträgt.

8. Hüftpfanne nach einem der Ansprüche 1 bis 7, **dadurch gekennzeichnet, dass** sich der Oberflächenabschnitt (6) mit dem Wandungsabschnitt (7) des äußeren Pfannenabschnitts (2) und dem Wandungsabschnitt (8) des inneren Pfannenabschnitts (4) vom unteren Ende (E) aus über einen Winkel (α) erstreckt, der zwischen 25° und 35° beträgt, wobei der Winkel (α) zwischen einer Linie, die vom unteren Ende des Wandungsabschnitt (7) des äußeren Pfannenabschnitts (2) senkrecht auf die Mittenachse (M) läuft und mit dieser einen Schnittpunkt bildet, und einer Linie, die vom genannten Schnittpunkt zum oberen Ende des Hohlraums (9) verläuft, gemessen wird.

9. Hüftpfanne nach Anspruch 8, **dadurch gekennzeichnet, dass** der Winkel (α) zwischen 28° und 32° beträgt.

10. Hüftpfanne nach einem der Ansprüche 1 bis 9, **dadurch gekennzeichnet, dass** die Länge (L) des Hohlraums (9), gemessen in Richtung der Mittenachse (M), zwischen 6,0 mm und 15,0 mm beträgt, bevorzugt zwischen 8,5 mm und 13,5 mm.

11. Hüftpfanne nach einem der Ansprüche 1 bis 10, **dadurch gekennzeichnet, dass** die radiale Dicke (C1) des massiven Abschnitts (11) des äußeren Pfannenabschnitts (2) beim Fortschreiten von seinem dem Pol (P) zugewandten Ende in Richtung zum unteren Ende (E) abnimmt.

## Claims

1. Acetabular cup (1) for a hip joint endoprosthesis, comprising an outer socket section (2) with a convex surface (3) for contact with the acetabulum and an inner socket section (4) with a concave surface (5) for contact with an inlay of the hip joint endoprosthesis, wherein at least over a surface section (6) of the acetabular cup (1), the outer socket section (2) is resiliently elastic relative to the inner socket section (4) in a radial direction (r), wherein the acetabular cup (1) has an upper pole (P) and a lower end (E) facing away from the pole (P), wherein the elasticity is provided by a wall section (7) of the outer socket section (2) which extends to a wall section (8) of the inner socket section (4) to form a cavity (9) with a radial distance (a), wherein the outer socket section (2) and the inner socket section (4) and the wall section (7) of the outer socket section (2) and the wall section (8) of the inner socket section (4) are formed integrally with one another, wherein the cavity (9) is free of spring elements, wherein the cavity (9) completely surrounds the circumference of the acetabular cup (1) and is open in the region of the lower end (E), wherein the outer socket section (2) has a radially outer porous structure (10) which is adjoined by a radially inner solid structure (11), wherein the outer porous structure (10) and the inner solid structure (11) are formed integrally with one another, wherein the wall section (7) of the outer socket section (2) has the radially outer porous structure (10) and wherein this adjoins a radially inner section with a solid structure (11),
**characterized in**
**that** the radially inner section with a solid structure (11) in the region of the lower end (E) has an L-shaped configuration (12) in cross-section, which closes off the radially outer porous structure (10) in the region of the lower end (E).

2. Acetabular cup according to claim 1, **characterised in that** the porous structure (10) is formed by a lattice, in particular by a cubic lattice.

3. Acetabular cup according to claim 1 or 2, **characterised in that** the radially outer porous structure (10) has a radial thickness (C2) and **in that** the radially inner section with a solid structure (11) has a radial thickness (C1), wherein the radial thickness (C1) of the solid structure (11) in the region of the lower end (E) is between 30 % and 50 % of the total radial thickness of the porous and solid structure.

4. Acetabular cup according to one of claims 1 to 3, **characterised in that** the radially inner section with a solid structure (11) at the end of the cavity (9) facing away from the lower end (E) has a thickness (D), measured perpendicular to the central axis (M), which is between 0.8 mm and 1.25 mm.

5. Acetabular cup according to one of claims 1 to 4, **characterised in that** the porous structure (10) has a porosity of at least 65 %, preferably at least 85 %.

6. Acetabular cup according to one of claims 1 to 5, **characterised in that** the wall section (7) of the outer socket section (2) has a radial thickness in the region of the surface section (6) and that the wall section (8) of the inner socket section (4) has a radial thickness (A), wherein the radial thickness (A) of the wall section (8) of the inner socket section (4) in the region of the lower end (E) is between 150 % and 250 % of the radial thickness of the wall section (7) of the outer socket section (2).

7. Acetabular cup according to one of claims 1 to 6, **characterised in that**, in the load-free state of the acetabular cup (1), in the region of the surface section (6) between the wall section (7) of the outer socket section (2) and the wall section (8) of the inner socket section (4) the cavity (9) has a radial thickness (B), wherein the radial thickness (B) of the cavity (9) in the region of the lower end (E) is between 5 % and 30 % of the radial thickness (A) of the wall section (8) of the inner socket section (4).

8. Acetabular cup according to one of claims 1 to 7, **characterised in that** the surface section (6) with the wall section (7) of the outer socket section (2) and the wall section (8) of the inner socket section (4) extends from the lower end (E) at an angle (α) of between 25° and 35°, wherein the angle (α) being measured between a line running perpendicularly from the lower end of the wall section (7) of the outer socket section (2) to the central axis (M) and forming an intersection with the latter, and a line running from the said intersection to the upper end of the cavity (9).

9. Acetabular cup according to claim 8, **characterised in that** the angle (α) is between 28° and 32°.

10. Acetabular cup according to one of claims 1 to 9, **characterised in that** the length (L) of the cavity (9), measured in the direction of the central axis (M), is between 6.0 mm and 15.0 mm, preferably between 8.5 mm and 13.5 mm.

11. Acetabular cup according to one of claims 1 to 10, **characterised in that** the radial thickness (C1) of the solid section (11) of the outer socket section (2) decreases as it progresses from its end facing the pole (P) towards the lower end (E).

## Revendications

1. Cupule acétabulaire (1) d'une endoprothèse de l'articulation de la hanche, comprenant une section extérieure (2) de cupule avec une surface convexe (3) destinée à venir en contact avec l'acétabulum et avec une section intérieure (4) de cupule avec une surface concave (5) destinée à venir en contact avec un insert de l'endoprothèse de l'articulation de la hanche, la section extérieure (2) de cupule étant configurée de manière élastique dans une direction radiale (r) par rapport à la section intérieure (4) de cupule au moins par une section de surface (6) de la cupule acétabulaire (1), la cupule acétabulaire (1) comportant un premier pôle (P) et une extrémité inférieure (E) opposée au pôle (P), l'élasticité étant assurée par une section de paroi (7) de la section extérieure (2) de cupule, qui s'étend à une distance radiale (a) par rapport à une section de paroi (8) de la section intérieure (4) de cupule en formant une cavité (9), la section extérieure (2) de cupule et la section intérieure (4) de cupule ainsi que la section de paroi (7) de la section extérieure (2) de cupule et la section de paroi (8) de la section intérieure (4) de cupule étant formées entre elles d'un seul tenant, la cavité (9) étant sans éléments de ressort, la cavité (9) s'étendant totalement tout autour de la périphérie de la cupule acétabulaire (1) et étant ouverte dans la zone de l'extrémité inférieure (E), la section extérieure (2) de cupule comportant une structure (10) poreuse située à l'extérieur radialement, à laquelle se raccorde une structure solide (11) située à l'intérieur radialement, la structure poreuse (10) située à l'extérieur et la structure solide (11) située à l'intérieur radialement étant formées l'une avec l'autre d'un seul tenant, la section de paroi (7) de la section extérieure (2) de cupule comportant la structure poreuse (10) située à l'extérieur radialement et celle-ci se raccordant à une section située à l'intérieur radialement avec une structure solide (11),
caractérisée en ce
la section située à l'intérieur radialement avec une structure solide (11) comporte, dans la zone de l'extrémité inférieure (E), en section, une forme en L (12), par laquelle la structure poreuse (10) située à l'extérieur radialement se termine dans la zone de l'extrémité inférieure (E).

2. Cupule acétabulaire selon la revendication 1, **caractérisée en ce que** la structure poreuse (10) est formée par un réseau, en particulier par un réseau cubique.

3. Cupule acétabulaire selon la revendication 1 ou 2, **caractérisée en ce que** la structure poreuse (10) située à l'extérieur radialement présente une épaisseur radiale (C2), et que la section située à l'intérieur radialement avec une structure solide (11) présente une épaisseur radiale (C1), l'épaisseur radiale (C1) de la structure solide (11) se situant dans la zone de l'extrémité inférieure (E) étant comprise entre 30 % et 50 % de la totalité de l'épaisseur radiale de la structure poreuse et de la structure solide.

4. Cupule acétabulaire selon l'une des revendications 1 à 3, **caractérisée en ce que** la section située à l'intérieur radialement avec une structure solide (11) présente, sur l'extrémité de la cavité (9) opposée à l'extrémité inférieure (E), une épaisseur (D), mesurée perpendiculairement à l'axe médian (M), qui est comprise entre 0,8 mm et 1,25 mm.

5. Cupule acétabulaire selon l'une des revendications 1 à 4, **caractérisée en ce que** la structure poreuse (10) présente une porosité d'au moins 65 %, de préférence d'au moins 85 %.

6. Cupule acétabulaire selon l'une des revendications 1 à 5, **caractérisée en ce que**, dans la zone de la section de surface (6), la section de paroi (7) de la section extérieure (2) de cupule présente une épaisseur radiale, et que la section de paroi (8) de la section intérieure (4) de cupule présente une épaisseur radiale (A), l'épaisseur radiale (A) de la section de paroi (8) de la section intérieure (4) de cupule dans la zone de l'extrémité inférieure (E) étant comprise entre 150 % et 250 % de l'épaisseur radiale de la section de paroi (7) de la section extérieure (2) de cupule.

7. Cupule acétabulaire selon l'une des revendications 1 à 6, **caractérisée en ce que**, dans l'état sans charge de la cupule acétabulaire (1), la cavité (9) avec une épaisseur radiale (B) est présente dans la zone de la section de surface (6) entre la section de paroi (7) de la section extérieure (2) de cupule et la section de paroi (8) de la section intérieure (4) de cupule, l'épaisseur radiale (B) de la cavité (9) dans la zone de l'extrémité inférieure (E) étant comprise entre 5 % et 30 % de l'épaisseur radiale (A) de la section de paroi (8) de la section intérieure (4) de cupule.

8. Cupule acétabulaire selon l'une des revendications 1 à 7, **caractérisée en ce que** la section de surface (6) avec la section de paroi (7) de la section extérieure (2) de cupule et la section de paroi (8) de la section intérieure (4) de cupule s'étend depuis l'extrémité inférieure (E) sur un angle (α) qui est compris entre 25° et 35°, l'angle (α) entre une ligne, qui s'étend depuis l'extrémité inférieure de la section de paroi (7) de la section extérieure (2) de cupule perpendiculairement à l'axe médian (M) et forme avec celui-ci un point d'intersection, et une ligne, qui s'étend depuis ledit point d'intersection jusqu'à l'extrémité supérieure de la cavité (9), étant mesuré.

9. Cupule acétabulaire selon la revendication 8, **caractérisée en ce que** l'angle (α) est compris entre 28° et 32°.

10. Cupule acétabulaire selon l'une des revendications 1 à 9, **caractérisée en ce que** la longueur (L) de la cavité (9), mesurée en direction de l'axe médian (M), est comprise entre 6,0 mm et 15,0 mm, de manière préférée entre 8,5 mm et 13,5 mm.

11. Cupule acétabulaire selon l'une des revendications 1 à 10, **caractérisée en ce que** l'épaisseur radiale (C1) de la section solide (11) de la section extérieure (2) de cupule diminue à mesure qu'elle avance depuis son extrémité opposée au pôle (P) en direction de l'extrémité inférieure (E).
